(19) Europäisches Patentamt
European Patent Office
Office européen des brevets

(11) Veröffentlichungsnummer : **0 111 329**
**B1**

(12) **EUROPÄISCHE PATENTSCHRIFT**

(45) Veröffentlichungstag der Patentschrift : **15.03.89**

(51) Int. Cl.⁴ : **C 07 D215/18**, A 61 K 31/47

(21) Anmeldenummer : **83112412.8**

(22) Anmeldetag : **09.12.83**

(54) **Fluormethylchinolin-Derivate und ihre Herstellung.**

(30) Priorität : **11.12.82 HU 400382**

(43) Veröffentlichungstag der Anmeldung : **20.06.84 Patentblatt 84/25**

(45) Bekanntmachung des Hinweises auf die Patenterteilung : **15.03.89 Patentblatt 89/11**

(84) Benannte Vertragsstaaten : **BE CH DE FR GB LI LU NL**

(56) Entgegenhaltungen :
**EP-A- 0 000 483**
**EP-A- 0 049 776**
**DE-A- 2 458 146**
**FR-A- 2 166 196**
**US-A- 2 432 393**
**JOURNAL OF HETEROCYCLIC CHEMISTRY, Band 2, Nr. 2, Juni 1965, Seiten 113-119. A. STEINACKER et al.: "Synthesis and properties of fluorine-containing heterocyclic compounds. I. Triflouromethyl quinolines (1)"**
**JOURNAL OF MEDICINAL CHEMISTRY, Band 11, Nr. 2, 26. Februar 1968, Seiten 267-269. R.M. PINDER et al.: "Antimalarials. II. Alpha-(2-Piperidyl)- and alpha-(2-Pyridyl)-2-trifluoromethyl-4-quinolinemethanols"**
**JOURNAL OF MEDICINAL CHEMISTRY, Band 14, Nr. 12, Dezember 1971, Seiten 1221-1222: J.C. CRAIG et al.: "Potential antimalarials. 7. Tribromomethylquinolines and positive halogen compounds"**
**GURNOS JONES: "Quinolines", Teil I, Seiten 735-738, John Wiley & Sons, London, GB**
**Hudlicky: Chemistry of Organic Flourme Compounds, Ellis Harwood Ltd (Wiley), 1976, 95-106**
**J. Fluorme Chemistry 14 (1979) 455-466**

(73) Patentinhaber : **ALKALOIDA VEGYESZETI GYAR**
**Postfach 1**
**H-4440 Tiszavasvári (HU)**

(72) Erfinder : **Salamon, Zoltán, Dipl.-Chem.**
**Elmunkás u. 11**
**Tiszavasvári (HU)**
Erfinder : **Imre geb. Virág, Ilona**
**Kabay J. u. 25**
**Tiszavasvári (HU)**
Erfinder : **Sebestyén, Magdolna**
**Irányi u. 16e**
**Hajdunánás (HU)**

(74) Vertreter : **Patentanwälte Beetz sen. - Beetz jun. Timpe - Siegfried - Schmitt-Fumian**
**Steinsdorfstrasse 10**
**D-8000 München 22 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 111 329 B1

Jouve, 18, rue St-Denis, 75001 Paris, France

Advances in Heterocyclic Chemistry, Academic Press, 1981, 2-5
Industrial and Engineering Chemistry 39(3), (1947) 389-391
Abstract eines Vortrages von Z.L. Salamon und J. Jekö auf dem IXth Symposium on the Chemistry of Heterocyclic Compounds, Bratislava/CSSR, 1987

**Beschreibung**

In jüngster Zeit gewannen fluorhaltige Chinolinderivate sprunghaft an Bedeutung. So sind zum Beispiel gegen Malaria wirksame Derivate (J. Med. Chem. 11 (1968) 267 und 14 (1971) 926 sowie US-A-4 429 130, DE-B-24 58 146 und DE-B-28 06 909) und Derivate mit analgetischer Wirksamkeit (Chem. Ther. 1973, 2 154-168) bekanntgeworden. Bei der Herstellung dieser Verbindungen stellen 4-Halogen-(tri)fluormethylchinolinderivate wichtige Zwischenprodukte dar. Die Erfindung betrifft neue Trichlormethylchinolinderivate sowie ein neues, wirtschaftliches und auch großtechnisch gut durchführbares Verfahren zur Herstellung von bekannten und neuen Fluormethylchinolinderivaten.

Gemäß der Literatur wurden derartige Verbindungen bisher wie folgt hergestellt.

Zur Herstellung aus fluorhaltigen Ausgangsverbindungen, zum Beispiel aus Trifluormethylanilin und Ethyltrifluoracetacetat, werden im wesentlichen die klassischen Chinolinsynthesen angewandt (J. Het. Chem. 2 (1965) 113 ; J. Med. Chem 11 (1968) 267 ; J. Het. Chem. 9 (1972) 1403-5).

Diese Verfahren ergeben im allgemeinen geringe bis mittelmäßige Ausbeuten. Die den Trifluormethylsubstituenten enthaltenden Ausgangsstoffe sind ferner relativ teuer. Infolge der nur kleinen bis mittelmäßigen Ausbeuten fallen außerdem erhebliche Mengen schwer vernichtbarer Fluorabfälle an, die eine Belastung für die Umwelt darstellen.

Es ist ferner auch möglich, Jod- bzw. Bromchinoline unter den Bedingungen der Ullmann-Reaktion in Gegenwart von Kupferstaub mit Trifluorjodmethan zu Trifluormethylchinolinen umzusetzen (Tetrahedron Lett. 1969 4095 ; Chem. Pharm. Bull. 18 (1970) 2334-9).

Auch hierbei werden nur kleine bis mittelmäßige Ausbeuten erzielt, und die Reaktion muß bei hohen Temperaturen vorgenommen werden. Das Reagens Trifluormethyljodid ist schließlich ein schwer zu handhabendes Gas, dessen Herstellung umständlich ist. Einigermaßen befriedigende Ausbeuten werden nur mit dem teuren, schwer herstellbaren Jodchinolin erreicht.

In ähnlicher Weise können aus den zumeist teuren, schwer herzustellenden Chinolincarbonsäuren (zum Beispiel aus den im homoaromatischen Ring carboxylsubstituierten Derivaten) durch Umsetzung mit Schwefeltetrafluorid in verflüssigtem Fluorwasserstoff Trifluormethylchinoline hergestellt werden (Chem. Pharm. Bull. 17 (1969) 2335-2339). Das Verfahren führt jedoch zu ziemlich schlechten Ausbeuten und ist durch die Verwendung des Schwefeltetrafluorids umständlich.

Ausgehend von Trichlormethyl- bzw. Tribrommethylchinolin ist nach bekannten Fluorierungsverfahren bis jetzt lediglich eine einzige Trifluormethylchinolinverbindung, das 2-Trifluormethylchinolin, hergestellt worden (US-A-2 432 393, Fluorierung gemäß Bull. Acad-Roy. Belg. 35 (1898) 375-420). Die Herstellungsbedingungen, die Ausbeute und die physikalischen Konstanten des Produkts sind in der Veröffentlichung nicht angegeben.

Der Erfindung liegt die Aufgabe zugrunde, neue Trichlormethylchinolinderivate sowie ein einfaches Verfahren zur Herstellung von Fluormethylchinolinderivaten daraus anzugeben, das gute Ausbeuten liefert und ohne teure Ausgangsverbindungen auskommt.

Die Aufgabe wird gemäß den Patentansprüchen 1 und 2 gelöst. Vorteilhafte Weiterbildungen sind Gegenstand der abhängigen Ansprüche.

Die erfindungsgemäßen Trichlormethylchinolinderivate besitzen die allgemeine Formel I

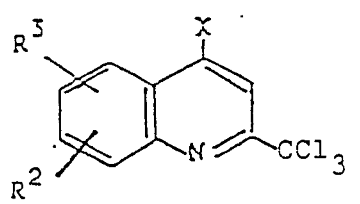

(I)

worin bedeuten :

$R^2$ Wasserstoff, Fluor, Chlor, Brom, Methyl oder $-CCl_3$,

$R^3$ Wasserstoff, Fluor, Chlor, Brom, Methyl oder $-CCl_3$ und

X Chlor oder Brom.

Das erfindungsgemäße Verfahren zur Herstellung von Fluormethylchinolinderivaten der Formel I',

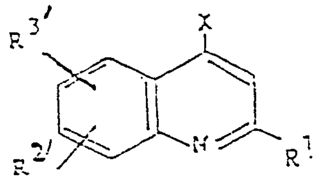

(I')

worin bedeuten :

$R^1$ $-CF_3$, $-CF_2Cl$, $-CFCl_2$ oder $-CCl_3$,

$R^{2'}$ Wasserstoff Fluor, Chlor, Brom, Methyl, $—CF_3$ oder $—CF_2Cl$ mit der Maßgabe, daß mindestens einer der Substituenten $R^1$ und $R^{2'}$ eine Trihalogenmethylgruppe mit wenigstens einem Fluoratom darstellt,

$R^{3'}$ Wasserstoff, Fluor, Chlor, Brom, Methyl oder $—CF_3$ und

X Chlor oder Brom,

ist gekennzeichnet durch Fluorierung von Trichlormethylchinolinderivaten der allgemeinen Formel I wie oben definiert mit Fluorwasserstoff in Gegenwart von Antimonpentachlorid oder Antimon(V)-fluorid in Gegenwart von Dichlormethan als Lösungsmittel bei einer Temperatur von — 20 bis + 30 °C oder in einem Gemisch aus Antimon(III)/Antimon(V)-fluoridchlorid bei einem Verhältnis Antimon(III) : Antimon(V) von 1 : 1 bis 5 : 1, einem Verhältnis Fluor : Chlor oder Brom von 0,8 bis 3,0 und einem Fluorgehalt des Reagens von 1,1 bis 2,8 Äquivalent, bezogen auf die zu ersetzenden Halogenatome, bei 40 bis 180 °C.

Die Ausgangsverbindungen des erfindungsgemäßen Verfahrens, d. h. die Trihalogenmethylchinoline, sind durch Chlorieren der einfach und aus billigen Rohstoffen herstellbaren Methylchinoline in guter Ausbeute erhältlich.

Als Fluorierungsmittel können auch Antimonfluoride verwendet werden. Bei Verwendung von Antimon(V)-fluorid wird vorzugsweise in Gegenwart von Dichlormethan als Lösungsmittel bei Temperaturen zwischen — 20 und + 30 °C gearbeitet. Das Fluorid wird, bezogen auf die zu ersetzenden Chlor- oder Bromatome der Halogenmethylgruppen des Trichlormethylchinolinderivats der Formel I in einer Menge von 0,25 bis 0,38 mol eingesetzt.

Besonders gute Ausbeuten werden erreicht, wenn man die Reaktion bei Temperaturen zwischen — 5 und 0 °C vornimmt. Diese Verfahrensweise hat den Vorteil, daß man das Reaktionsgemisch mit trockenem Fluorwasserstoff sättigen und auf diese Weise das Antimonfluorid regenerieren kann, wodurch das Verfahren noch wirtschaftlicher wird.

Erfindungsgemäß kann die Fluorierung auch mit gemischten Fluoriden, zum Beispiel mit Antimon(III)- und Antimon(V)-fluoridchlorid, vorgenommen werden, wobei das Molverhältnis zwischen Antimon(III) und Antimon(V) zu 1 : 1 bis 5 : 1 und das Verhältnis zwischen Fluor einerseits und Chlor oder Brom andererseits zu 0,8 bis 3,0 gewählt wird und das Reagens 1,1 bis 2,8 mol Fluor pro Mol zu ersetzende Halogenatome enthält. Diese Verfahrensvariante wird bei einer Reaktionstemperatur von 40 bis 180 °C und vorzugsweise 100 bis 160 °C durchgeführt.

Das Fluorierungsmittel kann zum Beispiel durch Vermischen von Antimon(III)-fluorid mit Antimon(V)-chlorid oder durch Umsetzung von Antimon(III)-fluorid mit der berechneten Menge Chlor oder auch durch Umsetzung von Chlor und Fluorwasserstoff mit Antimon(III)-chlorid hergestellt werden.

Das Verhältnis zwischen Antimon(III) und Antimon(V) bzw. zwischen Fluor und Chlor beeinflußt die Reaktion. Bei Einhaltung der oben angegebenen Verhältnisse wird die Kernhalogenierung zurückgedrängt und eine Teerbildung vermieden. Das Produkt läßt sich ferner leichter isolieren, wobei auch die Ausbeute besser ist.

Für die industrielle Nutzung des Verfahrens ist von besonderer Bedeutung, daß die gewünschte Umsetzung auch mit Fluorwasserstoff als Fluorierungsmittel in guter Ausbeute verläuft.

Auf 1 mol umzusetzendes Chinolinderivat werden als Fluorquelle 2 bis 10 Äquivalente und vorzugsweise 3 bis 5 Äquivalente trockener Fluorwasserstoff sowie 0,001 bis 3,5 mol und zweckmäßig 0,3 bis 0,6 mol Antimonpentachlorid eingesetzt. Die Umsetzung wird gegebenenfalls in Gegenwart eines Lösungsmittels bei Temperaturen von — 10 bis + 180 °C und vorzugsweise 60 bis 120 °C vorgenommen.

Aus EP-A-49776 sind 2-Trifluormethylchinolinderivate bekannt, die in 4-Stellung eine mit einer stickstoffhaltigen Gruppe substituierte Methylgruppe aufweisen und Antimalariawirkung besitzen. Diese Verbindungen werden aus entsprechenden 2-Trifluormethyl-4-halogenchinolinderivaten hergestellt.

DE-A-2458146 betrifft 2-Trifluormethylchinolinderivate, die in 4-Stellung mit einer Chinuclidingruppe substituiert sind und als Antimalariamittel verwendet werden können. Die Herstellung dieser Verbindungen erfolgt stets mit Chinolinderivaten als Ausgangsverbindungen, in denen bereits die Fluorsubstituenten enthalten sind.

In J. Med. Chem. 11 (2) 1968, 267-269, sind 2-Piperidyl-2-trifluormethyl-4-chinolinmethanole mit Antimalariawirkung beschrieben, die ausgehend von 2-Trifluormethylchinolinderivaten synthetisiert werden.

Chinolinderivate mit Antimalariawirksamkeit sind ferner in J. Med. Chem. 14 (12) 1971, 1221-1222, beschrieben, die in 2-Stellung eine Tribrommethyl-, Dibrommethyl- oder Trichlormethylgruppe aufweisen oder in 2-Stellung unsubstituiert sind, in 4-Stellung ein Chloratom tragen können und keine Fluoratome im Molekül enthalten.

In US-A-2432393 sind im Zusammenhang mit der Herstellung neuer Azofarbstoffe Azoverbindungen beschrieben, die als Kupplungskomponente eingeführte Trifluormethyl-1,2,3,4-tetrahydrochinolingruppen enthalten.

In der Monographie « Quinolines », part I, vol. 32, der Reihe « The Chemistry of Heterocyclic Compounds », G. Jones, ed., Wiley & Sons, sind auf den Seiten 735-738 zahlreiche Halogenchinoline beschrieben, die in 2-Stellung Halogenmethylgruppen, in 3-Stellung Halogenatome, in 4-Stellung Chlor, in 6-Stellung Halogen, Trifluormethyl oder Methyl und in 8-Stellung Methyl oder Halogenmethyl als Substituenten aufweisen können, wobei Einzelverbindungen mit bestimmten Substituentenkombinationen aufgelistet sind.

Gegenüber diesen vorgeschriebenen Verbindungen stellen die erfindungsgemäßen Verbindungen der Formel I eine erfinderische Auswahl dar.

J. Het. Chem. 2 (2) 1965, 113-119, sind substituierte 2-Trifluormethyl-4-chinolinole und ihre Herstellung zu entnehmen, die aus Ausgangsverbindungen mit entsprechender Fluorsubstitution am Chinolingerüst hergestellt werden. Auf die physiologische Wirksamkeit dieser Verbindungen aufgrund der Anwesenheit der Trifluormethylgruppe ist pauschal hingewiesen.

FR-A-2166196 betrifft schließlich 6,7-Dihydrobenzochinolizincarbonsäuren, die aus 1,2,3,4-Tetrahydrochinolinderivaten, die Trifluormethylgruppen aufweisen können, hergestellt werden. Diese Ausgangs- und Endprodukte unterscheiden sich strukturell von den Verbindungen gemäß der Erfindung.

Das erfindungsgemäße Verfahren wird im folgenden anhand von Beispielen näher erläutert.

## Beispiel 1

2-(Fluordichlormethyl)-8-methyl-4-chlorchinolin

Zu einer Lösung von 3,0 g 2-Trichlormethyl-8-methyl-4-chlorchinolin in 45 ml wasserfreiem Dichlormethan werden bei — 5 bis 0 °C unter intensivem Rühren zuerst 1,5 ml Antimonpentachlorid und dann 1,0 ml Antimonpentafluorid tropfenweise zugegeben. Das Gemisch wird bei der angegebenen Temperatur 4 Stunden lang gerührt und dann bei Raumtemperatur 48 Stunden lang stehengelassen. Dann wird das Gemisch mit 6 ml eiskalter konzentrierter Salzsäure versetzt ; der ausgefallene Niederschlag wird abfiltriert und mit wenig kaltem Dichlormethan nachgewaschen. Das mit der Waschflüssigkeit vereinigte Filtrat wird getrocknet, geklärt und eingedampft. Dabei werden 2,1 g festes Produkt erhalten. Nach Umkristallisieren aus Heptan gewinnt man 1,9 g einer gelben kristallinen Substanz. F. 90 bis 94 °C ; Ausbeute 68,2 %.

In ähnlicher Weise wird 2-(Fluordichlormethyl)-4,6,8-trichlorchinolin hergestellt ; F. 67 bis 72 °C.

## Beispiel 2

2-(Chlordifluormethyl)-8-methyl-4-chlorchinolin

Ein Gemisch von 3,6 g Antimontrifluorid und 0,5 ml Antimonpentachlorid wird unter Rühren auf 150 °C erwärmt, bei dieser Temperatur 5 Minuten gerührt und dann auf 115 °C abgekühlt. Bei dieser Temperatur setzt man dem Gemisch 2,95 g 2-Trichlormethyl-8-methyl-4-chlorchinolin zu und rührt bei 110 bis 120 °C eine Stunde lang. Das Reaktionsgemisch wird dann in eiskalte konzentrierte Salzsäure gegossen und mit Chloroform extrahiert. Die organische Phase wird getrocknet und eingedampft. Der Eindampfrückstand wird mit 4 × 50 ml Heptan extrahiert ; der Extrakt wird geklärt und eingedampft. Dabei werden 1,95 g eines später kristallisierenden Öls erhalten. F. 46-48 °C ; Ausbeute 74,4 %.

In ähnlicher Weise wird 2-(Chlordifluormethyl)-4,6,8-trichlorchinolin hergestellt ; F. 32 bis 34 °C.

## Beispiel 3

2-Trifluormethyl-8-methyl-4-chlorchinolin

Mit dem gemäß Beispiel 2 erhaltenen Gemisch von 3,6 g Antimontrifluorid und 0,5 ml Antimonpentachlorid werden 2,33 g 2-Trichlormethyl-4-chlor-8-methylchinolin umgesetzt. Die bei 150 bis 160 °C vorgenommene Umsetzung dauert 30 Minuten ; danach wird wie in Beispiel 2 aufgearbeitet. Hierbei werden 1,52 g Produkt in Form einer gelben Flüssigkeit erhalten, die durch Vakuumdestillation gereinigt werden kann. Kp. 155 bis 165 °C/24-29,3 mbar (155 bis 165 °C/18-22 Torr) ; Ausbeute 78,3 %.

Analog werden hergestellt :

| | F. (°C) |
|---|---|
| 2-Trifluormethyl-4,6,8-trichlorchinolin | 71-73 |
| 2-Trifluormethyl-4-chlorchinolin | 33-35 |
| 2-Trifluormethyl-6-methyl-4-chlorchinolin | 66-68 |
| 2-Trifluormethyl-6,8-dimethyl-4-chlorchinolin | 102-104 |
| 2-Trifluormethyl-4,8-dichlorchinolin | 58-59 |
| 2-Trifluormethyl-6-brom-4-chlorchinolin | 93-95 |
| 2-Trifluormethyl-6-fluor-4-bromchinolin | 96-98 |
| 2-Trifluormethyl-4,6-dichlorchinolin | 100,5-102,5 |
| 2-Trifluormethyl-5-methyl-4-chlorchinolin. | |

## Beispiel 4

2-Trichlormethyl-8-difluorchlormethyl-4-chlorchinolin

Ein Gemisch aus 2 g 2,8-Bis(trichlormethyl)-4-chlorchinolin, 4 ml wasserfreiem Fluorwasserstoff und 0,01 ml Antimonpentachlorid wird in einem geschlossenen Bombenrohr aus PTFE bei 100 °C 48 Stunden

lang umgesetzt. Dann wird das Gemisch in eine Mischung aus 100 g Eis und Natriumhydrogencarbonat gegossen und mit Chloroform (3 × 50 ml) extrahiert. Die organische Phase wird über Magnesiumsulfat getrocknet und dann eingedampft. Der Rückstand wird mit wenig Petrolether verrieben und dann filtriert. F. 71 bis 73 °C, Ausbeute 1,65 g.

Beispiel 5

2-Dichlorfluormethyl-8-trifluormethyl-4-chlorchinolin

Ein Gemisch aus 1 g 2,8-Bis(trichlormethyl)-4-chlorchinolin, 1,5 g Antimontrifluorid und 5 ml Chlorbenzol wird 18 Stunden lang am Rückfluß gekocht und dann abgekühlt. Das Gemisch wird danach zuerst mit 10 ml 10 %iger Salzsäure und dann mit 10 ml Wasser ausgeschüttelt, getrocknet, geklärt und eingedampft. Der Rückstand wird aus Petrolether umkristallisiert. F. 69 bis 71 °C ; Ausbeute 0,62 g.

Beispiel 6

2-Trichlormethyl-8-trifluormethyl-4-chlorchinolin

3,98 g 2,8-Bis(trichlormethyl)-4-chlorchinolin werden in 10 ml Dichlormethan gelöst. Zu der Lösung wird bei Raumtemperatur unter Rühren innerhalb von 2,5 Stunden ein Gemisch von 1 ml Antimonpentafluorid und 40 ml Dichlormethan tropfenweise zugegeben. Das Gemisch wird bei Raumtemperatur 4 Stunden lang gerührt und dann mit 50 ml 10-%iger Salzsäure versetzt. Nach Trennung der Phasen wird die organische Phase mit 50 ml Wasser gewaschen, über Natriumsulfat getrocknet und dann eingedampft. Der Eindampfrückstand (4,0 g) wird mit 3 × 50 ml Hexan heiß extrahiert, geklärt und dann eingedampft. Das Produkt wird aus 80-%igem Methanol umkristallisiert. F. 84 bis 86 °C ; Ausbeute 2,1 g.

Beispiel 7

2-Difluorchlormethyl-4-chlor-8-trifluormethylchinolin

Ein Gemisch aus 2 g 2,8-Bis(trichlormethyl)-4-chlorchinolin, 4 ml wasserfreiem Fluorwasserstoff und 0,1 ml Antimonpentachlorid wird 40 Stunden lang auf 150 °C gehalten und dann in ein Gemisch aus 100 g Eis und Natriumhydrogencarbonat gegossen und mit 3 × 50 ml Chloroform extrahiert. Die organische Phase wird über Magnesiumsulfat getrocknet und dann eingedampft. Der Rückstand wird mit Petrolether verrieben und dann filtriert. Die erhaltene Substanz (1,2 g) wird an einer Säule aus 40 g Kieselgel 60 mit Cyclohexan als Elutionsmittel chromatographiert. Durch Eindampfen der identischen Fraktionen erhält man 0,2 g 2-Fluordichlormethyl-4-chlor-8-trifluormethylchinolin und 0,7 g 2-Difluorchlormethyl-8-trifluormethyl-4-chlorchinolin ; F. 37 bis 39 °C.

Beispiel 8

2-Difluorchlormethyl-8-trifluormethyl-4-chlorchinolin

Eine Lösung von 11,8 g 2-Trichlormethyl-8-trifluormethyl-4-chlorchinolin in 30 ml absolutem Dichlormethan wird auf — 5 °C abgekühlt. Zwischen — 4 und — 6 °C werden innerhalb von 40 Minuten 1,8 ml Antimonpentafluorid zugetropft. Das Gemisch wird bei der angegebenen Temperatur noch 3,5 Stunden lang gerührt und dann mit 30 ml eiskalter konzentrierter Salzsäure vermischt. Die organische Phase wird noch mit 50 ml Wasser gewaschen, dann getrocknet, geklärt und eingedampft. Der Rückstand (11,1 g) wird mit 2 × 100 ml Heptan heiß extrahiert und das Heptan verdampft. Als Rückstand erhält man 8,3 g rohes Difluorchlormethyl-8-trifluormethyl-4-chlorchinolin. Nach Umkristallisieren aus wäßrigem Methanol bleiben 6,8 g Produkt ; F. 37-39 °C.

Beispiel 9

2,8-Bis(trifluormethyl)-4-chlorchinolin

Ein Gemisch aus 65 g Antimontrifluorid und 10 ml Antimonpentachlorid wird bei 150 °C 20 Minuten lang gerührt und dann auf 60 °C abgekühlt. Zu dem Reagens gibt man unter ständigem Kühlen 40 g 2,8-Bis(trichlormethyl)-4-chlorchinolin. Die Schmelze wird bei 60 bis 70 °C 5 Minuten lang gerührt und dann unter intensivem Rühren in 200 ml gekühlten Tetrachlorkohlenstoff eingegossen. Die Antimonhalogenide werden abfiltriert ; die organische Phase wird mit kalter Salzsäure gewaschen, getrocknet, geklärt und dann eingedampft. F. 40-42 °C ; Ausbeute 26,2 g.

Beispiel 10

2,8-Bis(trifluormethyl)-4-chlorchinolin

100 ml Dichlormethan werden auf — 10 °C abgekühlt. Zu dem Lösungsmittel gibt man 20 ml

Antimonpentafluorid und anschließend bei — 5 bis — 10 °C tropfenweise eine Lösung von 40 g 2,8-Bis(trichlormethyl)-4-chlorchinolin in 100 ml Dichlormethan. Das Gemisch wird 4 Stunden lang gerührt. Dann läßt man die Temperatur auf Raumtemperatur ansteigen und versetzt das Gemisch mit 700 ml 10-%iger Salzsäure. Man rührt 15 Minuten, trennt dann die Phasen voneinander und extrahiert die wäßrige Phase noch zweimal mit je 500 ml Dichlormethan. Die vereinigten organischen Phasen werden geklärt, getrocknet und dann eingedampft. Man erhält 32 g eines braunen Sirups, der durch Auflösen in 600 ml Hexan und Klären mit Aktivkohle gereinigt wird. F. 42 bis 44 °C ; Ausbeute 22,6 g.

### Beispiel 11

2,8-Bis(trifluormethyl)-4-chlorchinolin

In einen mit Rührer ausgerüsteten, druckfesten Autoklaven von 1 Liter Inhalt aus Hastelloy C werden 398,3 g 2,8-Bis(trichlormethyl)-4-chlorchinolin und 250 ml wasserfreier Fluorwasserstoff sowie 20 ml Antimonpentachlorid eingefüllt. Das Reaktionsgemisch wird unter ständigem Rühren und Abblasen von Chlorwasserstoffgas auf 170 bis 180 °C erwärmt. Das Gemisch wird dann bei dieser Temperatur 36 Stunden lang gerührt und danach abgekühlt, worauf der Fluorwasserstoff abdestilliert wird. Der Rückstand wird zwischen 1 Liter Dichlormethan und 500 ml Natriumcarbonatlösung verteilt. Die organische Phase wird mit 2 × 100 ml Wasser gewaschen, getrocknet und eingedampft. Der Rückstand wird durch Vakuumdestillation gereinigt. F. 45 bis 46 °C ; Kp. 148 bis 152 °C/24 mbar (148 bis 152 °C/18 Torr) ; Ausbeute 264,6 g.

### Beispiel 12

2,8-Bis(trifluormethyl)-4-chlorchinolin

39,83 g (0,1 mol) 2,8-Bis(trichlormethyl-4-chlorchinolin werden in 70 ml wasserfreiem Dichlormethan gelöst. Die Lösung wird unter ständigem Rühren auf — 5 °C abgekühlt. Zu der Suspension werden bei — 5 bis 0 °C innerhalb von 60 Minuten 10 ml Antimonpentafluorid tropfenweise zugegeben. Das Gemisch wird bei der angegebenen Temperatur noch 2 Stunden lang gerührt und dann mit eiskalter konzentrierter Salzsäure vermischt. Nach Trennung der Phasen wird die organische Phase mit Wasser gewaschen, getrocknet und dann eingedampft. Der Eindampfrückstand (30,6 g) wird im Vakuum fraktioniert. Die Hauptfraktion geht bei 150 bis 158 °C und 22,7 bis 25,3 mbar (17 bis 19 Torr) über. Das Destillat wird beim Stehen fest. F. 44 bis 46 °C ; Ausbeute 26,4 g (88,1 %).

Analog werden hergestellt :

2,7-Bis(trifluormethyl)-4-chlorchinolin, F. 35 bis 37 °C ;
2,6-Bis(trifluormethyl)-4-chlorchinolin, Flüssigkeit ;
2,5-Bis-(trifluormethyl)-4-chlorchinolin, Flüssigkeit ;
2,8-Bis(trifluormethyl)-4,5-dichlorchinolin, F. 52 bis 54 °C.

### Beispiel 13

Nach Beispiel 6 werden folgende Verbindungen hergestellt :

2-Trichlormethyl-4-chlor-6-trifluormethylchinolin, F. 46 bis 48 °C ;
2-Trichlormethyl-4-chlor-7-trifluormethylchinolin, F. 70 bis 73 °C ;
2-Trichlormethyl-4-chlor-5-trifluormethylchinolin, F. 63 bis 67 °C ;
2-Trichlormethyl-4-brom-8-trifluormethylchinolin, F. 73 bis 75 °C.

In der folgenden Tabelle sind physikalische Konstanten für erfindungsgemäße Verbindungen aufgeführt.

Die NMR-Spektren wurden in $CDCl_3$ aufgenommen ; bei den angegebenen Werten ist die erste Stelle nach dem Komma geschätzt. Die Verschiebungen sind auf Tetramethylsilan als Standard bezogen.

Verwendete Abkürzungen :

s Singulett
d Doublett
t Triplett
q Quartett
m Multiplett
b breites Signal

$J^x$ Kopplungskonstante, wobei der obere Index (z. B. x) die Anzahl der Bindungen angibt, über die die Kopplung wirkt. Bei heteronuklearer Kopplung weist der Index rechts unten auf die Kopplung, zum Beispiel F-H, hin.

Verbindung der Formel I *) :

| X | $R^3$ | $R^2$ | $^1H$-NMR-Daten (ppm) |
|---|---|---|---|
| Cl | H | $CClF_2$ | 8.16 ; d ($J^4$ 7.5 Hz ; 1H) 5H ; 8.05 d ($J^4_{F\text{-}H}$ 1Hz, 1H) 3H ; 7.93 d ($J^4$ 7.5 Hz, 1H) 7H. |

Verbindungen der Formel I' :

| $R^1$ | X | $R^3$ | $R^{2'}$ | $^1H$-NMR-Daten (ppm) |
|---|---|---|---|---|
| $CF_3$ | Cl | 5-$CH_3$ | H | 8.09 d ($J^3$, 7.6 Hz ; 1H) 3H ; 7.77 s (1H) 3H ; 7.68 dd ($J^3$ 7.6 Hz ; $J^{3'}$ 7.6 Hz ; 1H) 7H ; 7.52 d(b) ($J^3$ 7.6 Hz ; 1H) 6H ; 3.05 s (3H) Ho (5). |
| $CF_3$ | Cl | H | 7-$CH_3$ | 8.55 s(b) (1H) ; 8H ; 8.41 d ($J^3$ 8.5 Hz ; 1H) 5H ; 7.92 s (1H) 3H ; 7.91 d(b) ($J^3$ 8.5 Hz ; 1H) 6H. |
| $CF_3$ | Cl | H | 8-$CF_3$ | 8.47 dd ($J^3$ 8.5 Hz ; $J^4$ 1.5 Hz, 1H) 5H ; 8.21 dd(b) ($J^3$ 7.5 Hz ; $J^4$ 1.5 Hz ; 1H) 7H ; 7.90 s (1H) 3H ; 7.81 dd ($J^3$ 8.5 Hz ; $J^{3'}$ 7.5 Hz ; 1H) 6H. |
| $CCl_3$ *) | Cl | H | $CClF_2$ | 8.62 dd ($J^3$ 8.5 Hz ; $J^4$ 1.5 Hz ; 1H) 5H ; 8.50 s (1H) 3H ; 8.37 dd ($J^3$ 7.5 Hz ; $J^4$ 1.5 Hz ; 1H) 7H ; 8.05 dd ($J^3$ 7.5 Hz ; $J^3$ 8.5 Hz, 1H) 6H. |
| $CCl_2F$ | Cl | 8-Cl | 6-Cl | 8.16 ; d ($J^4$ 7.5 Hz ; 1H) 5H ; 8.05 d ($J^4_{F\text{-}H}$ 1Hz, 1H) 3H ; 7.93 d ($J^4$ 7.5 Hz, 1H) 7H. |
| $CCl_2F$ *) | Cl | H | 8-$CF_3$ | 8.62 dd (b) ($J^3$ 8.5 Hz ; $J^4$ 1.5 Hz ; 1H) 3H ; R.42 ddq ($J^3$ 7.5 Hz, $J^4$ 1.5 Hz ; $J^4_{F\text{-}H}$ 0.7 Hz 1H) 7H ; H.23 d ($J^4_{F\text{-}H}$ 0.73 Hz, 1H) 3H ; 8.05 ddq ($J^3$ 7.3 Hz, $J^{3'}$ 7.5 Hz ; $J^5_{F\text{-}H}$ 0.7 Hz) 6H ; |
| $CClF_2$ | Cl | 8-Cl | 6-Cl | 8.14 d ($J^4$ 2.0 Hz ; 1H) 3H ; 7.91 d ($J^4$ 2Hz, 1H) 7H ; 7.89 s (b) (1H) 3H. |
| $CClF_2$ | Cl | 8-$CH_3$ | H | 8.10 d (b) ($J^3$ 8.0 Hz ; 1H) 5H ; 7.80 s (1H) 3H ; 7.70 (b) ($J^3$ 8.0 Hz, 1H ; 7H ; 7.60 dd ($J^3$ 8.0 Hz ; $J^{3'}$ 8.0 Hz ; 1H) 6H ; 2.80 s (3H) S-Methyl. |

*) NMR-Spektrum in $CDCl_3$

**Patentansprüche**

1. Chinolinderivate der allgemeinen Formel I,

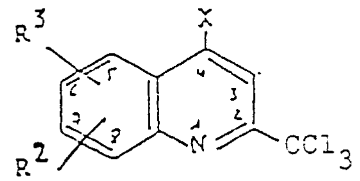

(I)

worin bedeuten :

$R^2$ Wasserstoff, Fluor, Chlor, Brom, Methyl oder $-CCl_3$,

$R^3$ Wasserstoff, Fluor, Chlor, Brom, Methyl oder $-CCl_3$ und

X Chlor oder Brom.

2. Verfahren zur Herstellung von Fluormethylchinolinderivaten der Formel I',

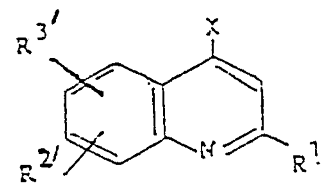

(I')

worin bedeuten :

$R^1$ $-CF_3$, $-CF_2Cl$, $-CFCl_2$ oder $-CCl_3$,

$R^{2'}$ Wasserstoff, Fluor, Chlor, Brom, Methyl, $-CF_3$ oder $-CF_2Cl$, mit der Maßgabe, daß mindestens einer der Substituenten $R^1$ und $R^{2'}$ eine Trihalogenmethylgruppe mit wenigstens einem Fluoratom darstellt,

$R^{3'}$ Wasserstoff, Fluor, Chlor, Brom, Methyl oder $-CF_3$ und

X Chlor oder Brom,

gekennzeichnet durch Fluorierung von Chinolinderivaten der allgemeinen Formel I,

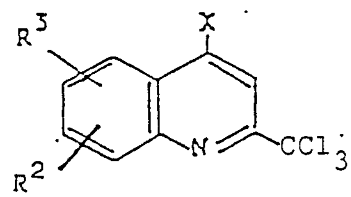

(I)

worin bedeuten :

$R^2$ Wasserstoff, Fluor, Chlor, Brom, Methyl oder $-CCl_3$,

$R^3$ Wasserstoff, Fluor, Chlor, Brom, Methyl oder $-CCl_3$ und

X Chlor oder Brom,

mit Fluorwasserstoff in Gegenwart von Antimonpentachlorid als Katalysator oder Antimon(V)-fluorid in Gegenwart von Dichlormethan als Lösungsmittel bei einer Temperatur von — 20 bis + 30 °C oder in einem Gemisch aus Antimon(III)/Antimon(V)-fluoridchlorid bei einem Verhältnis Antimon(III) : Antimon(V) von 1 : 1 bis 5 : 1 (vgl. S. 6, Z. 2), einem Verhältnis Fluor : Chlor oder Brom von 0,8 bis 3,0 und einem Fluorgehalt des Reagens von 1,1 bis 2,8 Äquivalent, bezogen auf die zu ersetzenden Halogenatome, bei 40 bis 180 °C.

3. Verfahren nach Anspruch 2, dadurch gekennzeichnet, daß das Fluorierungsmittel in einer Menge von 0,25 bis 0,38 mol, bezogen auf die zu ersetzenden Chlor- oder Bromatome des Chinolinderivats der allgemeinen Formel I, eingesetzt wird.

4. Verfahren nach Anspruch 2 oder 3, dadurch gekennzeichnet, daß das Antimon(V)-fluorid durch Behandeln des Reaktionsgemischs mit trockenem Fluorwasserstoffgas regeneriert wird.

5. Verfahren nach Anspruch 2, dadurch gekennzeichnet, daß als Fluorierungsmittel und Lösungsmittel trockener Fluorwasserstoff in einer Menge von 2 bis 10 Äquivalent, bezogen auf die zu ersetzenden Halogenatome, verwendet wird.

6. Verfahren nach Anspruch 2 oder 5, dadurch gekennzeichnet, daß der Katalysator in einer Menge von 0,001 bis 3,5 mol pro Mol Fluormethylchinolinderivat der allgemeinen Formel I eingesetzt wird.

## Claims

1. Quinoline derivatives of general formula I,

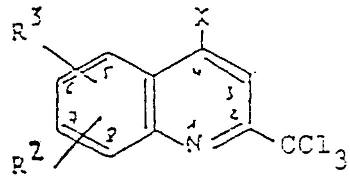

(I)

wherein
$R^2$ represents hydrogen, fluorine, chlorine, bromine, methyl or $—CCl_3$,
$R^3$ represents hydrogen, fluorine, chlorine, bromine, methyl or $—CCl_3$, and
X represents chlorine or bromine.

2. Process for preparing fluoromethylquinoline derivatives of formula I',

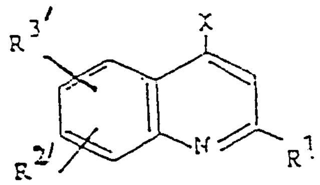

(I')

wherein
$R^1$ represents $—CF_3$, $—CF_2Cl$, $—CFCl_2$ or $—CCl_3$,
$R^{2'}$ represents hydrogen, fluorine, chlorine, bromine, methyl, $—CF_3$ or $—CF_2Cl$, with the proviso that at least one of the substituents $R^1$ and $R^{2'}$ represents a trihalomethyl group having at least one fluorine atom,
$R^{3'}$ represents hydrogen, fluorine, chlorine, bromine, methyl or $—CF_3$ and
X represents chlorine or bromine,
characterised by fluorination of quinoline derivatives of general formula I

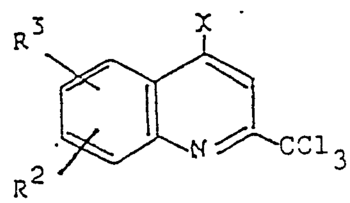

(I)

wherein
$R^2$ represents hydrogen, fluorine, chlorine, bromine, methyl or $—CCl_3$,
$R^3$ represents hydrogen, fluorine, chlorine, bromine, methyl or $—CCl_3$, and
X represents chlorine or bromine,
with hydrogen fluoride in the presence of antimony pentachloride as catalyst or antimony(V) fluoride in the presence of dichloromethane as solvent at a temperature of from — 20 to + 30 °C or in a mixture of antimony(III)/antimony(V) fluoridechloride in a ratio of antimony(III) to antimony(V) of 1 : 1 to 5 : 1 (see page 6, line 2), a ratio of fluorine to chlorine or bromine of 0.8 to 3.0 and a fluorine content of the reagent of 1.1 to 2.8 equivalents, based on the halogen atoms which are to be replaced, at 40 to 180 °C.

3. Process as claimed in claim 2, characterised in that the fluorination agent is used in a quantity of from 0.25 to 0.38 mole, based on the chlorine or bromine atoms of the quinoline derivative of general formula I which are to be replaced.

4. Process as claimed in claim 2 or 3, characterised in that the antimony(V) is regenerated by treating the reaction mixture with dry hydrogen fluoride gas.

5. Process as claimed in claim 2, characterised in that as the fluorination agent and solvent dry hydrogen fluoride is used in a quantity of 2 to 10 equivalents, based on the halogen atoms to be replaced.

6. Process as claimed in claim 2 or 5, characterised in that the catalyst is used in a quantity of from 0.001 to 3.5 mole per mole of fluoromethylquinoline derivative of general formula I.

## Revendications

1. Dérivés de quinoléine de formule générale I,

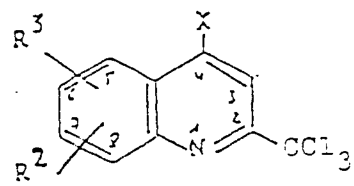

(I)

dans laquelle

$R^2$ représente l'hydrogène, le fluor, le chlore, le brome, un méthyle ou $-CCl_3$,

$R^3$ représente l'hydrogène, le fluor, le chlore, le brome, un méthyle ou $-CCl_3$, et

X représente le chlore ou le brome.

2. Procédé de préparation de dérivés de fluorométhylquinoléine de formule I',

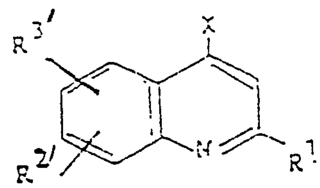

dans laquelle

$R^1$ représente $-CF_3$, $-CF_2Cl$, $-CFCl_2$ ou $-CCl_3$,

$R^{2'}$ représente l'hydrogène, le fluor, le chlore, le brome, un méthyle, $-CF_3$ ou $-CF_2Cl$, sous réserve qu'au moins l'un des substituants $R^1$ et $R^{2'}$ représente un groupe trihalogénométhyle avec au moins un atome de fluor,

$R^{3'}$ représente l'hydrogène, le fluor, le chlore, le brome, un méthyle ou $-CF_3$,

X représente le chlore ou le brome,

caractérisé par la fluoration de dérivés de quinoléine de formule générale (I),

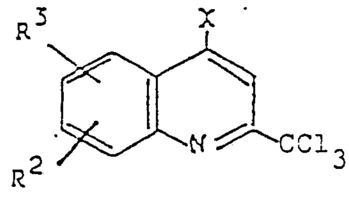

(I)

dans laquelle

$R^2$ représente l'hydrogène, le fluor, le chlore, le brome, un méthyle ou $-CCl_3$,

$R^3$ représente l'hydrogène, le fluor, le chlore, le brome, un méthyle ou $-CCl_3$, et

X représente le chlore ou le brome,

avec l'acide fluorhydrique en présence de pentachlorure d'antimoine comme catalyseur ou avec du fluorure d'antimoine(V) en présence de dichlorométhane comme solvant, à une température allant de — 20 à + 30 °C ou dans un mélange de fluorure chlorure d'antimoine(III)/antimoine(V) dans un rapport antimoine(III) : antimoine(V) allant de 1 : 1 à 5 : 1, dans un rapport fluor : chlore ou brome allant de 0,8 à 3,0 et avec une teneur en fluor du réactif allant de 1,1 à 2,8 équivalent, rapportée aux atomes d'halogène à substituer, à une température allant de 40 à 180 °C.

3. Procédé selon la revendication 2, caractérisé en ce que l'agent de fluoration est ajouté en une quantité allant de 0,25 à 0,38 mole, rapportée aux atomes de chlore ou de brome du dérivé de quinoléine de formule générale I à substituer.

4. Procédé selon la revendication 2 ou 3, caractérisé en ce que le fluorure d'antimoine(V) est régénéré en traitant le mélange réactionnel par de l'acide fluorhydrique gazeux sec.

5. Procédé selon la revendication 2, caractérisé en ce que l'on utilise de l'acide fluorhydrique sec, comme agent de fluoration et solvant, en une quantité allant de 2 à 10 équivalents, rapportée aux atomes d'halogène à substituer.

6. Procédé selon la revendication 2 ou 5, caractérisé en ce que le catalyseur est ajouté en une quantité allant de 0,001 à 3,5 mole par mole de dérivé de fluorométhylquinoléine de formule générale I.